# EUROPEAN PATENT SPECIFICATION

(11) **EP 2 376 476 B1**
(45) Date of publication and mention of the grant of the patent: **25.06.2014**
(21) Application number: 09795447.3
(22) Date of filing: 22.12.2009
(51) Int. Cl.: C07D 401/12

(54) **PROCESS FOR THE PREPARATION OF ESOMEPRAZOLE**
VERFAHREN ZUR HERSTELLUNG VON ESOMEPRAZOL
PROCÉDÉ DE SYNTHÈSE D'ÉSOMÉPRAZOLE

(30) Priority: 24.12.2008 SI 200800322; 24.06.2009 SI 200900172
(43) Date of publication of application: 19.10.2011
(73) Proprietor: KRKA, tovarna zdravil, d.d., Novo mesto, 8000 Novo mesto (SI)
(72) Inventor: KOTAR-JORDAN, Berta, 8311 Kostanjevica na Krki (SI); ZUPET, Rok, 1211 Ljubljana (SI); SMRKOLJ, Matej, 1420 Trbovlje (SI)
(74) Representative: UEXKÜLL & STOLBERG
(86) International application number: PCT/EP2009/067743
(87) International publication number: WO 2010/072759

(56) References cited:
- WO-A1-2006/094904
- WO-A1-2007/013743
- WO-A1-2008/004245

## Description

### FIELD OF THE INVENTION

The present invention relates to an improved process for preparation of esomeprazole by optical resolution of omeprazole with BINOL (1,1'-bi-2-naphthol).

### BACKGROUND OF THE INVENTION

Omeprazole, and its therapeutically acceptable alkaline salts are disclosed in EP 5129 and EP 124 495, respectively.

The optical resolution of omeprazole using a chiral acyl group, such as mandeloyl, is described in WO 94/27988. DE 40 35 455 discloses the separation of the enantiomers of omeprazole using diastereomeric ethers. The optical purification of enantiomerically enriched enriched omeprazole using a crystallization of the racemate is described in WO 97/02261. The resolution of omeprazole by bioreduction is described in WO 96/17077 and an enantioselective preparation of omeprazole by biooxidation is described in WO 96/17076. IPCOM000126473D discloses an optical resolution process using N-benzyl cinchoninium.

There have also been described processes for the optical resolution of omeprazole via an inclusion complex with BINOL. According to CN 1 223 262A the inclusion complex is formed in a solvent mixture comprising the toxic solvent benzene, and WO 2008/004245 discloses use of the toluene which also is a harmful solvent. WO 2008/149204 discloses the optical resolution of omeprazole using BINOL in the presence of aromatic or polyaromatic phenols. WO 2006/094904 describes formation of the BINOL inclusion complex in a solvent and in the presence of an amine, such as triethyl amine. However, again the undesirable solvent toluene is employed. Finally, WO 2007/013743 discloses preparation of the inclusion complex in a solvent which contains a weak base, such as an amine. The weak base is used to prevent the complex from taking an undesired black color.

In addition to these problems, the omeprazole and its enantiomer esomeprazole are temperature sensitive compounds and should be kept at relatively low temperatures as otherwise significant decomposition can occur. Thus, the prior processes suffer form the problem that the esomeprazole S-BINOL complex is generally precipitated from a hot solution. This can pose a problem because of the sensitivity of esomeprazole to heating and higher temperatures.

### DETAILED DESCRIPTION OF THE INVENTION

It is therefore an object of the present invention to provide a process for preparation of esomeprazole through formation of optically highly pure BINOL inclusion complex which does not require use of harmful solvents and minimizes exposure to high temperatures.

This object is surprisingly solved by the process according to the invention.

The process according to present invention for the preparation of esomeprazole or a salt thereof comprises the following steps:
(a) preparing (i) a solution of omeprazole and BINOL enantiomer in a solvent containing low concentrations of alkali metal hydroxide or alcoholate, wherein the molar ratio between the omeprazole and the hydroxide or alcoholate is 1:0.005 to 1:0.7 or (ii) a solution of an alkali or earth alkaline metal salt of omeprazole and BINOL enantiomer in a solvent,
(b) precipitating esomeprazole BINOL complex by treating the solution with an antisolvent or cooling the reaction mixture to initiate crystallization,
(c) optionally filtering the precipitate,
(d) converting the esomeprazole BINOL complex to esomeprazole or a salt thereof.

In step (a) (i) low concentrations of alkali metal hydroxides or alcoholates acting as solubilising agent are added. The molar ratio between omeprazole and the solubilising agent is preferably 1:0.005 to 1:0.7 and more preferably from 1:0.01 to 1:0.10. It is preferred to use sodium or potassium hydroxide.

Suitable solvents can be selected from the group consisting of alcohols, water, halogenated hydrocarbons, esters, ethers, ketones, nitriles, sulfoxides, amides and/or any mixtures thereof. Preferably the solvent is alcohol, ketone, water, halogenated hydrocarbon and/or any mixtures thereof, more preferably the solvent is alcohol or a mixture of alcohol and water. The alcohol is in particular ethanol, propanol or butanol. The preferred ketone is acetone, and the preferred ether is dioxane.

Suitable antisolvent can be selected from the group consisting of hydrocarbons, esters, ethers, ketones and/or any mixtures thereof. Preferably the antisolvent is hydrocarbon such as hexane, heptane or cyclohexane, and more preferred heptane and/or cyclohexane.

(R)- or (S)-BINOL can be used, and preferably the (S)-BINOL is used as BINOL enantiomer.

The solution according to step (a) (i) is in particular prepared by mixing omeprazol and BINOL enantiomer in a solvent with addition of solubilising agent at temperatures from in particular about 0-70°C, preferably 10-55°C, and more preferred 20-35°C. The solution formed in step (a) (i) can be cooled or to the solution formed in step (a) (i) antisolvent can be added and the obtained suspension is usually stirred for about 0.5 to 10 hours, and preferably 2 to 5 hours. The V/V ratio between solvent and antisolvent can be from 0.1 to 10, and preferably is between 0.4 to 2.5, and most preferred between 0.6 to 1.6. If necessary, the esomeprazole-BINOL complex can be recrystallized with the same solvent or with a solvent/antisolvent combination.

In a further embodiment of the process according to the invention, in step (a) (ii) a solution of an alkali or earth alkaline metal salt of omeprazole and BINOL enantiomer in a solvent is prepared. It is preferred to use the magnesium salt of omeprazole. The preferred solvent and preferred temperature are as mentioned above for step (a)(i).

The process according to the invention provides for esomeprazole-BINOL complex having an optical purity (measured according to method disclosed in European Pharmacopeia 6.3 determining percentage of the (R)-isomer, impurity F), hereinafter also referred to as enantiomeric purity, of in particular more than 99.0%, and preferably more than 99.5%. The chemical purity (measured by HPLC according European Pharmacopeia 6.3) is in particular more than 99.0%, and preferably about 99.5%.

It is surprising that the process of the invention allows for preparation of the esomeprazole BINOL complex in such a high purity without the need for harmful solvents and at moderate temperatures. The complex can easily be separated by conventional measures such as filtering.

Esomeprazole can be recovered from the esomeprazole BINOL complex by any known process, such as the process disclosed in CN 1 223 262A, WO 2006/094904, WO 2007/013743 and WO 2008/004245.

It is preferred to recover the esomeprazole in step (d) of the process by treating the complex with an alkali metal hydroxide in a mixture of organic solvent and water. The hydroxide is preferably NaOH, and the organic solvent is preferably isopropyl acetate.

Esomeprazole obtained by the process of the present invention can be converted to any pharmaceutically acceptable salt, and preferably magnesium salt. The magnesium salt of esomeprazole obtained by the process of the present invention is preferably having from 0% to 1% of (R)-isomer content, and in particular from 0.01% to 0.5%.

The BINOL used in the process according to the invention can be recovered from the reaction mixture in particular by treating the organic layer after separation of esomeprazole with aqueous solution, followed by separation of the organic layer which is optionally treated with activated carbon. The BINOL may be recovered from the solution by conventional means and further recrystallized from a solvent such as alcohol, preferably ethanol.

Also described are pharmaceutical compositions comprising esomeprazole or its salts prepared according to the present invention. More specifically, pharmaceutical compositions are described comprising magnesium esomeprazole.

The pharmaceutical compositions may be prepared and stored in a packaging material usually chosen by those of ordinary skill in the art for maintaining stability of drugs. Decreased moisture permeability of primary packaging material is preferred in order to diminish moisture sorption by esomeprazole containing dosage forms to avoid any changes in drug stability. Low moisture permeable primary packaging materials such as polychloro-3-fluoroethylene homopolymer/PVC laminate can be used with a thickness in the range 270 µm to 360 µm. In case of Al/Al blisters laminate with a thickness in the range 100 µm to 155 µm can be used. Optionally, dosage forms containing esomeprazole or its pharmaceutically acceptable salt can be packed into primary packaging with desiccant. The desiccant can be placed inside the packaging unit together with esomeprazole dosage units such as tablets and/or capsules in the closure system or can be incorporated into the walls of the primary packaging unit.

According to a preferred embodiment, contact between the pharmaceutical composition and environmental oxygen may be reduced or suppressed by either packaging the pharmaceutical composition under reduced pressure, packaging in an inert gas atmosphere, using a coating affording protection and stability of the pharmaceutical composition to environmental influences, or by using a packaging wherein the contact between the pharmaceutical composition and oxygen is reduced by the means of oxygen absorbers.

In another embodiment the moisture content expressed as relative humidity RH of gas surrounding the solid composition such as a tablet and/or capsule inside the primary packaging can be reduced typically to below 45%, preferably below 40% and most preferably below 30%. For example, as packaging material blisters, glass bottles, containers made of polymeric materials with suitable closure systems may be used. Packaging can be performed under normal atmosphere or optionally under an atmosphere having a reduced oxygen concentration, preferably in an inert atmosphere and/or under reduced relative humidity such as 40% RH, preferably 35% RH and most preferably below 30% RH. Reduced oxygen concentration means that the concentration of oxygen in the gas surrounding the solid composition in the primary packaging is below 18 vol/vol%, preferably below 10 vol/vol% and most preferably below 5vol/vol%. Optionally dry nitrogen as an inert dry atmosphere can be used during packaging of the parmaceutical compositions.

An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon may be used, or by the use of absorbents. Suitable absorbents may be selected from the group of commercially available absorbents such as humidity-activated oxygen absorbers, ultraviolet-radiation-activated absorbers, radiation-activated absorbers, microwaves-radiation-activated absorbers, absorbers activated by a combination of activation processes or absorbers without necessity of activation. Examples of commercially available absorbers are Ageless™ (Mitsubishi Gas Chemical), ATCO (Standa Industry), FreshPax™ (Multisorb Technologies), O-Buster™ (Hsiao Sung Non-Oxygen Chemical Co), Biotika Oxygen Absorber (Biotika) and the like.

Also described are stabilized packaged pharmaceutical compositions comprising esomeprazole, wherein the packaging has a space for trapping and disposal of free oxygen. Moreover, if the active compounds of the present composition are exhibited to a reduced oxygen partial pressure, the composition is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Alpolychloro-3-fluoroethylene homopolymer/PVC laminate blister. The invention is in the following further illustrated by means of examples.

### EXAMPLES

### Preparation of esomeprazole-(S)-BINOL complex

### Example 1

The suspension of 50.0 g (0.1447 mole) omeprazole in 250 ml 96 % ethanol with addition of 0.46 g (0.0360 mole) potassium hydroxide is gradually added to the solution of 25 g (0.0870 mole) (S)-(-)-BINOL in 250 ml 95 % ethanol at 60-65°C. The obtained solution is slowly cooled to 20-25°C and maintained at this temperature for 5 hours; pH of the suspension is 9.9. The product is filtered, washed or reslurried with 25 ml of 95 % ethanol and finally washed with 25 ml of cyclohexane. The product is dried at 40°C to obtain 36.2 g (S)-omeprazole S-BINOL with enantiomeric purity of more than 99.5 %.

### Example 2

The suspension of 100.0 g (0.2895 mole) omeprazole in 500 ml 96 % ethanol with addition of 4.6 g (0.072 mole) potassium hydroxide is gradually added to the solution of 50 g (0.1746 mole) (S)-(-)-BINOL in 500 ml 95 % ethanol at 60-65°C. The obtained solution is slowly cooled to 30°C, the solution is seeded with 1 g of (S)-omeprazole-(S)-BINOL complex and maintained at 20-25°C for 10 hours, pH of the suspension is 10.0. The suspension is cooled to 0-5°C and stirred for 3 hours. The product is filtered, washed or reslurried with 25 ml of 95 % ethanol and finally washed with 700 ml 96% ethanol. The product is dried at 40°C to obtain 47.6 g (S)-omeprazole (S)-binol with enantiomeric purity of more than 99.5 %.

### Example 3

10.0 g (0.0289 mole) omeprazole is added to the solution of 5 g (0.0174 mole) (S) - (-) -BINOL in 80 ml 1-butanol, 20 ml water with addition of 0.29 g (0.00718 mole) sodium hydroxide at 60-65°C. The obtained solution is slowly cooled to 20-25°C and maintained at this temperature for 5 hours, pH of the suspension is 10.2. The product is filtered, washed or reslurried with 20 ml aqueous 1-butanol (4:1). The product is dried at 40°C to obtain 4.8 g (S)-omeprazole (S)-BINOL with an enantiomeric purity of more than 99.5 %.

### Example 4

10.0 g (0.0289 mole) omeprazole is added to the solution of 5 g (0.0174 mole) (S)-(-)-BINOL in 80 ml 1-propanol, 20 ml water with addition of 0.29 g (0.00718 mole) sodium hydroxide at 60-65°C. The obtained solution is slowly cooled to 20-25°C and maintained at this temperature for 5 hours, pH of the suspension is 9.9. The product is filtered, washed or reslurried with 20 ml aqueous 1-propanol (4:1). The product is dried at 40°C to obtain 4.6 g (S)-omeprazole (S)-BINOL with an enantiomeric purity of more than 99.5 %.

### Example 5

The solution of 51.6 g (0.0724 mole) omeprazole magnesium in 300 ml methanol is gradually added to the solution of 25 g (0.0870 mole) (S)-(-)-BINOL in 500 ml 95 % ethanol at 50-55°C. The obtained solution is concentrated in vacuo to half of volume (about 500 ml), then slowly cooled to 20-25°C and maintained at this temperature for 5 hours. The product is filtered, washed or reslurried with 25 ml of 95 % ethanol and finally washed with 25 ml of cyclohexane. The product is dried at 40°C to obtain 34.1g (S)-omeprazole (S)-BINOL with a chromatographic purity of more than 99.5 %.

### Example 6

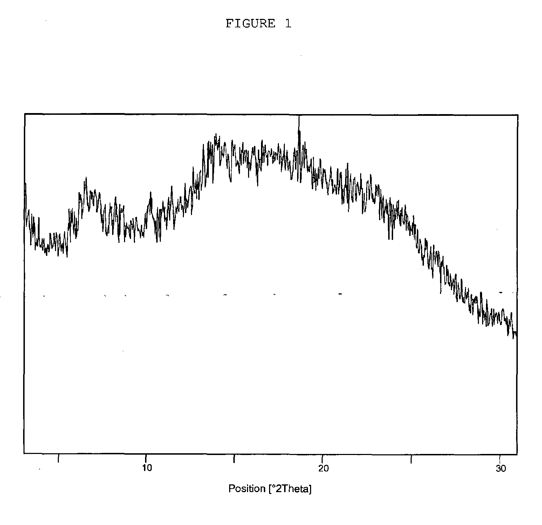

The suspension of 50.0 g (0.1447 mole) omeprazole in 200 ml acetone with addition of 0.46 g (0.0360 mole) potassium hydroxide is gradually added to the solution of 25 g (0.0870 mole) (S)-(-)-BINOL in 200 ml acetone at 60-65°C. The product is precipitated with 500 ml of hexane at 20-25°C and stirred at that temperature for 2 hours. The suspension is filtered and washed with 50 ml of hexane. The wet product is dissolved in 1000 ml of acetone, concentrated in vacuo to half of volume (about 500 ml) and precipitated with 600 ml of hexane at 20-25°C. The suspension is ageing for 2 hours. The product is washed with 50 ml of hexane and dried at 40°C to obtain 37.6 g esomeprazole (S)-binol with a chromatographic purity of more than 99.5 %. The X-ray powder diffraction pattern of the esomeprazole (S)-BINOL complex is shown in Figure 1.

### Example 7

The suspension of 50.0 g (0.1447 mole) omeprazole in 200 ml dioxane with addition of 0.46 g (0.0360 mole) potassium hydroxide is gradually added to the solution of 25 g (0.0870 mole) S-(-)-BINOL in 200 ml dioxane at 60-65°C. The product is precipitated with 500 ml of cyclohexane at 20-25°C and stirred at that temperature for 2 hours. The suspension is filtered and washed with 50 ml of cyclohexane. The wet product is dissolved in 1000 ml of dioxane, concentrated in vacuo to half of volume (about 500 ml) and precipitated with 600 ml of cyclohexane at 20-25°C. The suspension is ageing for 2 hours. The product is washed with 50 ml of cyclohexane and dried at 40°C to obtain 30.2 g (S)-omeprazole S-BINOL with a chromatographic purity of more than 99.9 %.

### Preparation of esomeprazole magnesium

### Example 8

100.0 g (0.1583 mole) (S)-omeprazole (S)-BINOL and 500 ml of isopropyl acetate were added to 500 ml aqueous solution containing 9.9 g NaOH at room temperature. The phases are separated and aqueous layer once again extracted with 500 ml of isopropyl acetate with adjusting pH to 12.0 ± 0.1. Aqueous layer is separated in two equal halves and magnesium salt of S-omeprazole is precipitated with:
a) 1.68 g ( 0.04155 mole) magnesium oxide at 20-25°C with stirring for 12 hours and adjusting pH to 10.0 ± 0.1. The product is filtered, washed or reslurried with 150 ml of water and dried at 40°C to obtain 20.8 g esomeprazole magnesium.
b) 100 ml aqueous solution of 3.96 g ( 0.04155 mole) magnesium chloride at 20-25°C with stirring for 4 hours and adjusting pH to 10.0 ± 0.1. The product is filtered, washed or reslurried with 150 ml of water and dried at 40°C to obtain 24.1 g esomeprazole magnesium.

### Example 9

100.0 g (0.1583 mole) (S)-omeprazole (S)-BINOL and 500 ml of isopropyl acetate were added to 500 ml aqueous solution containing 9.9 g NaOH at room temperature. The phases are separated and once again extracted with 500 ml of isopropyl acetate with adjusting pH to 12.0 ± 0.5. The aqueous layer is separated, 250 ml of methanol is added and the magnesium salt of (S)-omeprazole is precipitated with 100 ml aqueous solution of 7.92 g ( 0.0831 mole) magnesium chloride at 20-25°C with stirring for 4 hours. The product is filtered, washed or reslurried with 150 ml of water and dried at 40°C to obtain 50.0 g esomeprazole magnesium.

### (S)-omeprazole magnesium dihydrate as polymorphic form A is crystallized as follows:

50.0 g of (S)-omeprazole magnesium is dissolved in 500 ml of methanol at 25-30°C or filtered to get a clear solution. The solution is concentrated to 90-100 ml (assay 30-35%). The concentrated solution is cooled to 20 ± 2°C, 400 ml acetone/water mixture (4:1) is gradually added to the solution such that the temperature does not exceed 25°C. The suspension is stirred for 12 hours at 20 ± 2°C, then 3 hours at 0-5°C, filtered and washed with 50 ml of acetone. About 70 g of wet product is dried in a vacuum or an air dryer with rotation first at a temperature of from 20 to 35°C for about 6-10 hours following gradually for 10°C up to 60-70°C for 1-2 hours if it is necessary. Drying is controlled by Karl Fischer (KF), Loss on drying (LOD) and GC analyses.
Yield: 40.0 g (S)-omeprazole magnesium dihydrate as polymorphic form A

The particle size of two batches of the obtained esomeprazole Mg dihydrate form A were as follows:

| | **Batch 1** | **batch 2** |
|---|---|---|
| **mean particle size** | **12 µm** | **20 µm** |
| d₁₀ | 4.2 µm | 5.2 µm |
| d₅₀ | 10.0 µm | 14.2 µm |
| d₉₀ | 24.4 µm | 42.3 µm |
| **Specific surface area** | **5.5 m²/g** | **5.6 m²/g** |

The particle size was determined by laser light scattering method using e.g. a Malvern Mastersizer 2000 Apparatus with vegetable oil as the dilution medium. The specific surface area was measured by nitrogen adsorption, using 6 point Brunauer, Emmett and Teller (BET) method.

### Pharmaceutical compositions of magnesium esomeprazole:

The following examples describe pharmaceutical compositions which include esomeprazole prepared according to the process of the invention.

**These examples are background art and do not form part of the present invention.**

### Example 10

| **Ingredients** | **composition [mg]** | **percentage** |
|---|---|---|
| **Core material** | | |
| (S) - esomeprazole magnesium dihydrate form A | 41.29 | 31.94 % |
| Neutral spheres | 71.16 | 55.06% |
| Povidone | 15.00 | 11.61 % |
| Sodium lauryl sulphate | 1.80 | 1.39 % |

| **Separating layer** | | |
|---|---|---|
| Core material | 129.25 | 70.97 % |
| Opadry II White (PVA, talc, TiO2, Macrogol) | 46.88 | 25.74 % |
| Magnesium hydroxide carbonate heavy (I) | 6.00 | 3.29 % |

| **Enteric coating** | | |
|---|---|---|
| Pellets with separating layer | 182.13 | 60.65 % |
| Methacrylic acid ethyl acrylate copolymer | 76.49 | 25.7 % |
| Talc | 22.95 | 7.64 % |
| Macrogol | 7.65 | 2.55 % |
| Titanium dioxide | 7.65 | 2.55 % |
| Polysorbate 80 | 3.44 | 1.14 % |

A drug suspension was prepared by suspending the esomeprazole magnesium salt into water solution of sodium lauryl sulphate and by adding the water solution of povidone. The suspension was kept at 16°C during the layering process using the minimal agitation (minimal speed of mixing propeller). Suspension layering of neutral spheres (e.g. sugar or microcrystalline pellets) was performed in a fluid bed processor using bottom spray technique (Wurster column) at a batch size of 3.0 kg. The spray operation was stopped when the specified amount of bulk liquid had been sprayed.

The prepared core material was dried until the loss on drying of the pellets was about 1-1.5 %. The pellets were then covered with separating layer in a Wurster column. The suspension consisted of purified water, magnesium hydroxide carbonate heavy and Opadry powder mixture, the latter being composed of polyvinyl alcohol, talc, titanium dioxide and macrogol. Coated pellets were dried until the loss on drying was about 1-1.5 %. The enteric coating suspension was prepared using methacrylic acid ethyl acrylate copolymer (Eudragit L 30 D-55), macrogol, talc, titanium dioxide and Polysorbate 80. The suspension was sprayed onto the pellets covered with separating layer in a fluid bed apparatus. The spray operation was stopped when the specified amount of bulk liquid had been sprayed, and then drying was carried in the Wurster column.

Pellets were filled into hard gelatine capsules.

### Example 11

| **Ingredients** | **Composition [mg]** | **percentage** |
|---|---|---|
| **Core material** | | |
| (S) - omeprazole magnesium dihydrate form A | 41.29 | 31.94 % |
| Neutral spheres | 71.16 | 55.06% |
| Povidone | 15.00 | 11.61 % |
| Sodium lauryl sulphate | 1.80 | 1.39 % |

| **Separating layer 1** | | |
|---|---|---|
| Core material | 129.25 | 81.45 % |
| Opadry II White (PVA, talc, TiO2, Macrogol) (I) | 23.44 | 14.77 % |
| Magnesium hydroxide carbonate heavy | 6.00 | 3.78 % |

| **Separating layer 2** | | |
|---|---|---|
| Core material | 158.69 | 87.13 % |
| Opadry II White (PVA, talc, TiO2, Macrogol) (II) | 23.44 | 12.87 % |

| **Enteric coating** | | |
|---|---|---|
| Pellets with separating layer | 182.13 | 60.65 % |
| Methacrylic acid ethyl acrylate copolymer | 76.49 | 25.47 % |
| Macrogol | 22.95 | 7.64 % |
| Talc | 7.65 | 2.55 % |
| Titanium dioxide | 7.65 | 2.55 % |
| Polysorbate 80 | 3.44 | 1.14 % |

A drug suspension was prepared by suspending the esomeprazole magnesium salt into water solution of sodium lauryl sulphate and by adding the water solution of povidone. The suspension was kept at 16°C during the layering process. Suspension layering of neutral spheres (e.g. sugar or microcrystalline pellets) was performed in a fluid bed processor using bottom spray technique (Wurster column) at a batch size of 3.0 kg. The spray operation was stopped when the specified amount of bulk liquid had been sprayed.

The prepared core material was dried until the loss on drying of the pellets was about 1-1.5 %. The pellets were then covered with two separating layers in a Wurster column. Both suspensions consisted of purified water and Opadry powder mixture, the latter being composed of polyvinyl alcohol, talc, titanium dioxide and macrogol. The first suspension for separating layer additionally contained magnesium hydroxide carbonate heavy. Coated pellets were dried until the loss on drying was about 1-1.5 %.

The enteric coating suspension was prepared using methacrylic acid ethyl acrylate copolymer (Eudragit L 30 D-55), macrogol, talc, titanium dioxide and Polysorbate 80. The suspension was sprayed onto the pellets covered with separating layer in a fluid bed apparatus. The spray operation was stopped when the specified amount of bulk liquid had been sprayed, and then drying was carried in the Wurster column.

Pellets were filled into hard gelatine capsules.

### Example 12

| **Ingredients** | **composition [mg]** | **percentage** |
|---|---|---|
| **Core material** | | |
| (S) - omeprazole magnesium dihydrate form A | 41.29 | 31.94 % |
| Neutral spheres | 71.16 | 55.06% |
| Hypromellose | 15.00 | 11.61 % |
| Sodium lauryl sulphate | 1.80 | 1.39 % |

| **Separating layer 1** | | |
|---|---|---|
| Core material | 129.25 | 81.45 % |
| Opadry II White (PVA, talc, TiO2, Macrogol) (I) | 23.44 | 14.77 % |
| Magnesium hydroxide carbonate heavy | 6.00 | 3.78 % |

| **Separating layer 2** | | |
|---|---|---|
| Core material | 158.69 | 87.13 % |
| Opadry II White (PVA, talc, TiO2, Macrogol) (II) | 23.44 | 12.87 % |

| **Enteric coating** | | |
|---|---|---|
| Pellets with separating layer | 182.13 | 60.65 % |
| Methacrylic acid ethyl acrylate copolymer | 76.49 | 25.47 % |
| Macrogol | 22.95 | 7.64 % |
| Talc | 7.65 | 2.55 % |
| Titanium dioxide | 7.65 | 2.55 % |
| Polysorbate 80 | 3.44 | 1.14 % |

A drug suspension was prepared by suspending the esomeprazole magnesium salt into water solution of sodium lauryl sulphate and by adding the water solution of hypromellose. The suspension was kept at 16°C during the layering process. Suspension layering of neutral spheres (e.g. sugar or microcrystalline pellets) was performed in a fluid bed processor using bottom spray technique (Wurster column) at a batch size of 3.0 kg. The spray operation was stopped when the specified amount of bulk liquid had been sprayed.

The prepared core material was dried until the loss on drying of the pellets was about 1-1.5 %. The pellets were then covered with two separating layers in a Wurster column. Both suspensions consisted of purified water and Opadry powder mixture, the latter being composed of polyvinyl alcohol, talc, titanium dioxide and macrogol. The first suspension for separating layer additionally contained magnesium hydroxide carbonate heavy. Coated pellets were dried until the loss on drying was about 1-1.5 %.

The enteric coating suspension was prepared using methacrylic acid ethyl acrylate copolymer (Eudragit L 30 D-55), macrogol, talc, titanium dioxide and Polysorbate 80. The suspension was sprayed onto the pellets covered with separating layer in a fluid bed apparatus. The spray operation was stopped when the specified amount of bulk liquid had been sprayed, and then drying was carried in the Wurster column.

Pellets were filled into hard gelatine capsules.

### Example 13

| **Ingredients** | **composition [mg]** | **percentage** |
|---|---|---|
| **Core material** | | |
| (S) - omeprazole magnesium dihydrate form A | 41.29 | 32.45 % |
| Neutral spheres | 71.15 | 55.92 % |
| Povidone | 13.00 | 10.22 % |
| Sodium lauryl sulphate | 1.80 | 1.41 % |

| **Separating layer** | | |
|---|---|---|
| Core material | 127.24 | 89.77 % |
| Methylcellulose | 8.00 | 5.64 |
| Magnesium hydroxide carbonate heavy | 6.50 | 4.59 % |

| **Enteric coating** | | |
|---|---|---|
| Pellets with separating layer | 141.74 | 73.21 % |
| Methacrylic acid ethyl acrylate copolymer | 42.52 | 21.96 % |
| Glycerol monostearate | 2.13 | 1.10 % |
| Triethyl citrate | 6.38 | 3.29 % |
| Polysorbate 80 | 0.85 | 0.44 % |

A drug suspension was prepared by suspending the esomeprazole magnesium salt into water solution of sodium lauryl sulphate and by adding the water solution of povidone. The suspension was kept at 16°C during the layering process. Suspension layering of neutral spheres (e.g. sugar or microcrystalline pellets) was performed in a fluid bed processor using bottom spray technique (Wurster column) at a batch size of 3.0 kg. The spray operation was stopped when the specified amount of bulk liquid had been sprayed.

The prepared core material was dried until the loss on drying of the pellets was about 1-1.5 %. The pellets were then covered with the separating layer in a Wurster column. The suspension consisted of purified water, methylcellulose and magnesium hydroxide carbonate heavy. Coated pellets were dried until the loss on drying was about 1-1.5 %.

The enteric coating suspension was prepared using methacrylic acid ethyl acrylate copolymer (Eudragit L 30 D-55), triethyl citrate, glyceryl monostearate and Polysorbate 80. The suspension was sprayed onto the pellets covered with separating layer in a fluid bed apparatus. The spray operation was stopped when the specified amount of bulk liquid had been sprayed, and then drying was carried in the Wurster column. Pellets were filled into hard gelatine capsules.

## Claims

1. A process for the preparation of esomeprazole or a salt thereof, which comprises the following steps
(a) preparing (i) a solution of omeprazole and BINOL enantiomer in a solvent containing low concentrations of alkali metal hydroxide or alcoholate, wherein the molar ratio between the omeprazole and the hydroxide or alcoholate is 1:0.005 to 1:0.7 or (ii) a solution of an alkali or earth alkaline metal salt of omeprazole and BINOL enantiomer in a solvent,
(b) precipitating esomeprazole BINOL complex by treating the solution with an antisolvent or cooling the reaction mixture to initiate crystallization,
(c) optionally filtering the precipitate,
(d) converting the esomeprazole BINOL complex to esomeprazole or a salt thereof.

2. Process according to claim 1, wherein the alkali metal hydroxide is sodium or potassium hydroxide.

3. Process according to claim 1 or 2, wherein the BINOL enantiomer is (S)-BINOL.

4. Process according to any one of claims 1 to 3, wherein the solvent is alcohol, ketone, water, halogenated hydrocarbon or a mixture thereof, preferably alcohol or a mixture of alcohol and water.

5. Process according to any one of claims 1 to 4, wherein the antisolvent is hydrocarbon, preferably hexane, heptane or cyclohexane, and most preferred heptane and/or cyclohexane.

6. Process according to any one of claims 1 to 5, wherein the BINOL is recovered from the reaction mixture.

7. Process according to any one of claims 1 to 6, wherein the molar ratio between the omeprazole and the hydroxide or alcoholate is 1:0.01 to 1:0.10.

8. Process according to any one of claims 1 to 7, wherein step (a) is conducted at 0 to 70°C, preferably 10 to 55°C and most preferred 20 to 35°C.

9. Process according to any one of claims 1 to 8, wherein the ratio (V/V) between solvent and antisolvent is 0.1 to 10, preferably 0.4 to 2.5 and most preferred 0.6 to 1.6.

10. Process according to any one of claims 1 to 9, wherein the esomeprazole BINOL complex has an optical purity of more than 99.0% and preferably more than 99.5%.

11. Process according to any one of claims 1 to 10, wherein the salt of esomeprazole is magnesium esomeprazole.

12. Process according to any one of claims 1 to 11, wherein in step (a) (ii) the magnesium salt of omeprazole is used.

13. Process according to any one of claims 1 to 12, wherein in step (d) the esomeprazole BINOL complex is treated with an alkali metal hydroxide in a mixture of organic solvent and water.

## Patentansprüche

1. Verfahren zur Herstellung von Esomeprazol oder einem Salz davon, welches die folgenden Schritte beinhaltet,
(a) Herstellung (i) einer Lösung von Omeprazol und BINOL-Enantiomer in einem Lösungsmittel, das niedrige Konzentrationen von Alkalimetallhydroxid oder -alkoholat enthält, wobei das Molverhältnis zwischen dem Omeprazol und dem Hydroxid oder Alkoholat 1:0,005 bis 1:0,7 beträgt, oder (ii) einer Lösung eines Alkali- oder Erdalkalimetallsalzes von Omeprazol und BINOL-Enantiomer in einem Lösungsmittel,
(b) Ausfällen von Esomeprazol-BINOL-Komplex durch Behandlung der Lösung mit einem Antilösungsmittel oder Kühlen der Reaktionsmischung, um Kristallisation auszulösen,
(c) gegebenenfalls Abfiltrieren der Ausfällung,
(d) Umwandeln des Esomeprazol-BINOL-Komplexes zu Esomeprazol oder einem Salz davon.

2. Verfahren nach Anspruch 1, bei dem das Alkalimetallhydroxid Natrium- oder Kaliumhydroxid ist.

3. Verfahren nach Anspruch 1 oder 2, bei dem das BINOL-Enantiomer (S)-BINOL ist.

4. Verfahren nach einem der Ansprüche 1 bis 3, bei dem das Lösungsmittel Alkohol, Keton, Wasser, halogenierter Kohlenwasserstoff oder eine Mischung davon, vorzugsweise Alkohol oder eine Mischung von Alkohol und Wasser ist.

5. Verfahren nach einem der Ansprüche 1 bis 4, bei dem das Antilösungsmittel Kohlenwasserstoff, vorzugsweise Hexan, Heptan oder Cyclohexan und besonders bevorzugt Heptan und/or Cyclohexan ist.

6. Verfahren nach einem der Ansprüche 1 bis 5, bei dem das BINOL aus der Reaktionsmischung gewonnen wird.

7. Verfahren nach einem der Ansprüche 1 bis 6, bei dem das Molverhältnis zwischen dem Omeprazol und dem Hydroxid oder Alkoholat 1:0,01 bis 1:0,10 beträgt.

8. Verfahren nach einem der Ansprüche 1 bis 7, bei dem Schritt (a) bei 0 bis 70°C, vorzugsweise 10 bis 55°C und besonders bevorzugt 20 bis 35°C durchgeführt wird.

9. Verfahren nach einem der Ansprüche 1 bis 8, bei dem das Verhältnis (V/V) zwischen Lösungsmittel und Antilösungsmittel 0,1 bis 10, vorzugsweise 0,4 bis 2,5 und besonders bevorzugt 0,6 bis 1,6 beträgt.

10. Verfahren nach einem der Ansprüche 1 bis 9, bei dem der Esomeprazol-BINOL-Komplex eine optische Reinheit von mehr als 99,0 % und vorzugsweise mehr als 99,5 % hat.

11. Verfahren nach einem der Ansprüche 1 bis 10, bei dem das Salz von Esomeprazol Magnesium-Esomeprazol ist.

12. Verfahren nach einem der Ansprüche 1 bis 11, bei dem in Schritt (a) (ii) das Magnesiumsalz von Omeprazol verwendet wird.

13. Verfahren nach einem der Ansprüche 1 bis 12, bei dem in Schritt (d) der Esomeprazol-BINOL-Komplex mit einem Alkalimetallhydroxid in einer Mischung von organischem Lösungsmittel und Wasser behandelt wird.

## Revendications

1. Procédé de préparation de l'ésoméprazole ou de l'un de ses sels, lequel procédé comporte les étapes suivantes :
a) préparer
i) une solution d'oméprazole et d'un énantiomère de BINOL (1,1'-bis(2-naphtol)) dans un solvant contenant, en faible concentration, un hydroxyde ou un alcoolate de métal alcalin, dans laquelle le rapport molaire entre l'oméprazole et l'hydroxyde ou l'alcoolate vaut de 1/0,005 à 1/0,7,
ii) ou une solution d'un sel de métal alcalin ou alcalino-terreux d'oméprazole et d'un énantiomère de BINOL dans un solvant ;
b) faire précipiter un complexe d'ésoméprazole et de BINOL, en traitant la solution avec un anti-solvant ou en refroidissant le mélange réactionnel pour amorcer la cristallisation ;
c) en option, séparer par filtration le précipité ;
d) et convertir le complexe d'ésoméprazole et de BINOL en ésoméprazole ou en l'un de ses sels.

2. Procédé conforme à la revendication 1, dans lequel l'hydroxyde de métal alcalin est de l'hydroxyde de sodium ou de potassium.

3. Procédé conforme à la revendication 1 ou 2, dans lequel l'énantiomère de BINOL est le (S)-BINOL.

4. Procédé conforme à l'une des revendications 1 à 3, dans lequel le solvant est un alcool, une cétone, de l'eau, un hydrocarbure halogéné, ou un mélange de tels solvants, et de préférence, un alcool ou un mélange d'un alcool et d'eau.

5. Procédé conforme à l'une des revendications 1 à 4, dans lequel l'anti-solvant est un hydrocarbure, de préférence de l'hexane, de l'heptane ou du cyclohexane, et surtout de l'heptane et/ou du cyclohexane.

6. Procédé conforme à l'une des revendications 1 à 5, dans lequel on récupère le BINOL à partir du mélange réactionnel.

7. Procédé conforme à l'une des revendications 1 à 6, dans lequel le rapport molaire entre l'oméprazole et l'hydroxyde ou l'alcoolate vaut de 1/0,01 à 1/0,10.

8. Procédé conforme à l'une des revendications 1 à 7, dans lequel l'étape (a) est effectuée à une température de 0 à 70 °C, de préférence de 10 à 55 °C, et surtout de 20 à 35 °C.

9. Procédé conforme à l'une des revendications 1 à 8, dans lequel le rapport en volume entre le solvant et l'anti-solvant vaut de 0,1 à 10, de préférence de 0,4 à 2,5, et surtout de 0,6 à 1,6.

10. Procédé conforme à l'une des revendications 1 à 9, dans lequel le complexe d'ésoméprazole et de BINOL présente une pureté optique de plus de 99,0 %, et de préférence de plus de 99,5 %.

11. Procédé conforme à l'une des revendications 1 à 10, dans lequel le sel d'ésoméprazole est le sel de magnésium d'ésoméprazole.

12. Procédé conforme à l'une des revendications 1 à 11, dans lequel on utilise, dans l'étape (a-ii), du sel de magnésium d'oméprazole.

13. Procédé conforme à l'une des revendications 1 à 12, dans lequel, dans l'étape (d), on traite le complexe d'ésoméprazole et de BINOL avec un hydroxyde de métal alcalin, dans un mélange de solvant organique et d'eau.
